**Europäisches Patentamt**

**European Patent Office**

**Office européen des brevets**

(19)

(11) Veröffentlichungsnummer: **0 064 651**
**B1**

# (12) EUROPÄISCHE PATENTSCHRIFT

(45) Veröffentlichungstag der Patentschrift:
**20.06.84**

(21) Anmeldenummer: **82103489.9**

(22) Anmeldetag: **24.04.82**

(51) Int. Cl.³: **C 07 C 143/66**

(54) **Verfahren zur Isolierung von H-Säure und K-Säure.**

(30) Priorität: **07.05.81 DE 3118147**

(43) Veröffentlichungstag der Anmeldung:
**17.11.82 Patentblatt 82/46**

(45) Bekanntmachung des Hinweises auf die Patenterteilung:
**20.06.84 Patentblatt 84/25**

(84) Benannte Vertragsstaaten:
**CH DE FR GB IT LI**

(56) Entgegenhaltungen:
**EP - A - 0 009 092**
**EP - A - 0 009 793**
**FR - A - 2 386 520**

(73) Patentinhaber: **BAYER AG, Konzernverwaltung RP**
**Patentabteilung, D-5090 Leverkusen 1 Bayerwerk (DE)**

(72) Erfinder: **Behre, Horst, Dr., Zur alten Linde 12,**
**D-5068 Odenthal (DE)**
Erfinder: **Blank, Heinz Ulrich, Dr., Am Geusfelde 35,**
**D-5068 Odenthal (DE)**
Erfinder: **Lindner, Otto, Dr., Strässchen Siefen 31,**
**D-5060 Bergisch-Gladbach 2 (DE)**
Erfinder: **Schössler, Willi, Dr., Hahnenweg 2,**
**D-5000 Köln 80 (DE)**

ACTORUM AG

EP 0 064 651 B1

## Beschreibung

Die Erfindung betrifft ein verbessertes Verfahren zur Isolierung von H-Säure und K-Säure aus diese beiden Säuren enthaltenden Gemischen.

H-Säure (1-Amino-8-naphthol-3,6-disulfonsäure) und K-Säure (1-Amino-8-naphthol-4,6-disulfonsäure) sind wichtige Zwischenprodukte zur Herstellung von Farbstoffen (s. Ullmanns Enzyklopädie der Technischen Chemie, 4. Auflage, Band 17, Seite 106).

Nach den alten Verfahren zu ihrer Herstellung wurden die beiden Säuren durch alkalische Druckhydrolyse der ihnen entsprechenden Naphthylamintrisulfonsäuren gewonnen, H-Säure aus T-Säure (1-Naphthylamin-3,6,8-trisulfonsäure) und K-Säure aus Melansäure (1-Naphthylamin-4,6,8-trisulfonsäure).

Aus der DE-OS 28 43 680 ist ein Verfahren zur Herstellung der Monoalkalisalze der K-Säure bekannt, bei dem ein Gemisch isomerer Naphthylamintrisulfonsäuren mit Melansäure und T-Säure als Hauptbestandteilen und/oder deren Salze mit Alkalihydroxid-Lösungen bei erhöhtem Druck und erhöhter Temperatur umgesetzt und nach dem Ansäuern der alkalischen Reaktionslösungen H- und K-Säure als Monoalkalisalze entweder getrennt nacheinander durch fraktionierte Kristallisation bei verschiedenen Temperaturen oder als Gemisch bei einer Temperatur abgetrennt werden und das Gemisch anschliessend durch fraktioniertes Herauslösen der K-Säure in K- und H-Säure zerlegt wird.

Die in diesem Verfahren angewendete Isolierung von H- und K-Säure aus diese beiden Säuren und auch noch andere isomere Amino-naphthol-sulfonsäuren sowie eine Reihe weiterer Verbindungen enthaltenden Gemischen befriedigt jedoch noch nicht in Bezug auf Ausbeute und Reinheit der so erhaltenen H- und K-Säure.

Es wurde nun gefunden, dass sich sowohl die Ausbeuten als auch die Reinheit von H- und K-Säure bei ihrer Isolierung aus die beiden Säuren und gegebenenfalls noch andere isomere Amino-naphthol-sulfonsäuren enthaltenden Gemischen wesentlich verbessern lassen, wenn man H- und K-Säure, nicht wie bislang aus sauren Lösungen ausfällt, die nur die Ionen eines Alkalimetalles, nämlich nur Natrium- oder nur Kaliumionen enthalten, sondern wenn man die Fällung der H-Säure aus sauren Lösungen vornimmt, die sowohl Natrium- als auch Kaliumionen enthalten.

Die Erfindung betrifft daher ein Verfahren zur Isolierung von H-Säure und K-Säure in Form ihrer Monoalkalisalze aus diese beiden Säuren, Alkaliionen und gegebenenfalls andere Aminonaphthol-disulfonsäuren enthaltenden sauren wässrigen Lösungen durch Ausfällen der H-Säure-Monoalkalisalze bei höheren Temperaturen und Ausfällen der K-Säure-Monoalkalisalze durch Abkühlen des nach dem Abtrennen der H-Säure anfallenden Filtrates, das dadurch gekennzeichnet ist, dass man die H-Säure aus Lösungen ausfällt, die sowohl Natrium- als auch Kaliumionen enthalten.

Das Verhältnis von Natrium- und Kaliumionen beträgt in den sauren Lösungen vorteilhaft 5-1:1, vorzugsweise 3-2:1.

Die Gesamtkonzentration der sauren Lösungen an Alkaliionen beträgt für die Fällung der H-Säure vorteilhaft 1–10 g Atome/1 Lösung, vorzugsweise 3–8 g-Atome/1 Lösung.

Durch das Ausfällen der H-Säure aus sauren Lösungen, die sowohl Natrium- als auch Kaliumionen enthalten, wird die H-Säure in Form gemischter Mononatrium-Monokalium-Salze ausgefällt. Überraschenderweise weisen diese gemischten Natrium-Kalium-Salze der H-Säure eine wesentlich geringere Löslichkeit als die Mononatrium- und Monokaliumsalze der H-Säure auf. Infolge der Schwerlöslichkeit der gemischten Mononatrium-Monokalium-Salze der H-Säure fällt nicht nur die H-Säure annähernd quantitativ und in hoher Reinheit aus der H- und K-Säure enthaltenden Lösung aus, sondern zugleich wird auch die K-Säure in höheren Ausbeuten und höherer Reinheit erhalten, da beim Ausfällen der K-Säure keine die Ausfällung beeinträchtigende H-Säure mehr in der Lösung vorhanden ist.

Gegenüber dem in der DE-OS 2 843 680 beschriebenen Verfahren zur Isolierung von H-Säure und K-Säure wird für H-Säure eine Ausbeutesteigerung von 62 auf 70% und für K-Säure eine Ausbeutesteigerung von 70 auf 75 Gew.-% erreicht. Ausserdem werden H- und K-Säure nach dem erfindungsgemässen Verfahren in höherer Reinheit erhalten.

Zum Abscheiden der H-Säure in Form ihrer gemischten Mononatrium-Monokaliumsalze werden die die H- und K-Säure und gegebenenfalls noch andere Amino-naphthol-disulfonsäuren enthaltenden Lösungen durch Zugabe von Mineralsäuren auf einen pH-Wert von 0 bis 4, vorzugsweise 0,5 bis 2,5 eingestellt.

Die H-Säure wird vorzugsweise aus sauren wässrigen Lösungen ausgefällt, deren Feststoffgehalt (Summe aus Amino-Naphthol-disulfonsäuren und Alkalisalzen) etwa 10 bis 60 Gew.-%, vorzugsweise 20 bis 50 Gew.-% beträgt.

Der Feststoffgehalt der Filtrate, aus denen die K-Säure ausgefällt wird, beträgt vorteilhaft 10 bis 60 Gew.-%, vorzugsweise 20 bis 50 Gew.-%.

Das Ausfällen der H-Säure aus den sauren Lösungen in Form ihrer gemischten Mononatrium-Monokalium-Salze wird bei Temperaturen von 20 bis 100°C, vorzugsweise 30 bis 90°C, insbesondere 40 bis 60°C, vorgenommen. Die ausgefällte H-Säure wird mechanisch, z.B. durch Filtrieren oder Schleudern, von der Lösung abgetrennt. Das abgetrennte Produkt wird mit wässriger alkalisulfatlösung und/oder mit Wasser gewaschen und anschliessend, gegebenenfalls im Vakuum, getrocknet. Die Waschlaugen werden im folgenden Ansatz anstelle von Wasser wiederverwendet.

Die K-Säure wird aus den nach dem Abtrennen der H-Säure-Monoalkalisalze anfallenden sauren Filtraten ausgefällt. Dies geschieht in der Weise, dass man das saure Filtrat, nachdem man den Gehalt an Natriumionen gegebenenfalls durch Zugabe von Natriumionen abgebenden Verbindungen, z.B. von Natriumsalzen wie Kochsalz oder Natriumsulfat, oder Natriumhydrogensulfat, auf eine Konzentration von 1 bis 10 g-Atome/l Filtrat, vorzugsweise 2 bis 6 g-Atome/l Filtrat eingestellt hat, auf eine Temperatur unterhalb der Fällungstemperatur der H-Säure abkühlt. Je nach dem, bei welcher Temperatur die H-Säure ausgefällt wurde, wird die K-Säure bei Temperaturen von −5 bis 40°C, vorzugsweise 10 bis 30°C, insbesondere 15 bis 25°C, ausgefällt. Nach beendeter Fällung wird das K-Säuremonoalkalisalz mechanisch von der Lösung abgetrennt, z.B. durch Filtrieren oder Schleudern. Das abgetrennte Produkt wird mit wässriger Natriumsulfat- oder Natriumchloridlösung und/oder Wasser gewaschen und anschliessend, gegebenenfalls im Vakuum, getrocknet. Die Waschlaugen werden im folgenden Ansatz anstelle von Wasser wiederverwendet.

Die erfindungsgemässen Verhältnisse von Natriumionen zu Kaliumionen in den zum Ausfällen der H-Säure bestimmten sauren Lösungen können auf verschiedene Weise eingestellt werden. Im einfachsten Fall dadurch, dass man den bei der Druckhydrolyse der Naphthylamintrisulfonsäuren mit Natriumhydroxid oder Kaliumhydroxid anfallenden alkalischen Lösungen vor, während oder nach dem Ansäuern mit Mineralsäuren die fehlende Alkaliionenart in Form diese Alkaliionen abgebender Verbindungen zusetzt, z.B. Kaliumionen, vorzugsweise in Form von Kaliumsalzen wie Kaliumchlorid, Kaliumsulfat und Kaliumhydrogensulfat, Natriumionen, vorzugsweise in Form von Natriumsalzen wie Natriumchlorid oder Natriumsulfat oder Natriumhydrogensulfat. Man kann jedoch das erfindungsgemässe Ionenverhältnis auch schon in einer früheren Verfahrensphase dadurch einstellen, dass man die Naphthylamintrisulfonsäuren und/oder deren Natrium- oder Kaliumsalze mit einer dem gewünschten Natrium-Kaliumionen-Verhältnis entsprechenden Mischung von Natronlauge und Kalilauge bzw. entsprechenden Mengen an Natronlauge oder Kalilauge hydrolysiert, so dass in dem Hydrolyse-Gemisch bereits das erfindungsgemässe Natrium/Kalium-Ionenverhältnis vorliegt.

Das erfindungsgemässe Verfahren zur Abtrennung von H-Säure und K-Säure aus diese beiden Säuren und gegebenenfalls noch andere Amino-naphthol-disulfonsäuren enthaltenden Lösungen lässt sich zur Isolierung von H- und K-Säuren aus Lösungen anwenden, die H- und K-Säure in den verschiedensten Gewichtsverhältnissen enthalten. Das Gewichtsverhältnis von H-Säure/K-Säure kann in den Lösungen zwischen 1:6 bis 6:1 schwanken.

Von besonderem Interesse ist die Isolierung von H- und K-Säure aus Lösungen, die, bezogen auf die in ihnen enthaltenen Amino-naphthol-sulfonsäuren, etwa 10 bis 70 Gew.-% K-Säure, 70 bis 10 Gew.-% H-Säure und etwa 1 bis 25 Gew.-% andere Amino-naphthol-disulfonsäuren sowie weitere quantitativ nicht bestimmbare Verbindungen aus den Vorstufen zur Herstellung der Amino-naphthol-disulfonsäuren-Isomerengemische enthalten. Solche Lösungen fallen bei der Herstellung von K-Säure durch alkalische Druckhydrolyse von Roh-Melansäure-Isomeren-Gemischen an wie sie in DE-OS 2 843 680 beschrieben sind oder auf beliebige andere Weise hergestellt werden können.

Beispiel 1

In einem 2,7 l Nickelautoklaven werden 761 g eines Naphthylamin-trisulfonsäure-Gemisches in Form der Trinatriumsalze (Gehalt 9,07 g Gesamtnitrit/100 g, 25,2 Gew.-% Melansäure MG 383; insgesamt 69 g Nitrit, 0,50 Mol Melansäure) folgender Zusammensetzung:

| | |
|---|---|
| 1-Naphthylamin-4,6,8-trisulfonsäure | 50,0% |
| 1-Naphthylamin-3,6,8-trisulfonsäure | 29,6% |
| 2-Naphthylamin-4,6,8-trisulfonsäure | 8,8% |
| 1-Naphthylamin-2,5,7-trisulfonsäure | 1,2% |
| 1-Naphthylamin-3,5,7-trisulfonsäure | 2,5% |
| 2-Naphthylamin-3,6,8-trisulfonsäure | 0,2% |

(%-Gehalte bezogen auf diazotierbare Substanz); das zusätzlich 12,1 Gew.-% Wasser und quantitativ nicht bestimmbare Mengen an Amino- und Nitroderivaten der Dinaphthylsulfon-sulfonsäuren und an Oxidationsprodukten des Naphthalins und der Naphthalin-trisulfonsäuren enthält, sowie 160 g (4,0 Mol) Natriumhydroxid, 140 g (2,5 Mol) Kaliumhydroxid und 608 g Wasser vorgelegt, wodurch, bezogen auf das gesamte Wasser, eine 30 gew.-%ige Alkalihydroxidlösung entsteht. Die Reaktionsmischung wird auf 170°C aufgeheizt, 12 Stunden bei 170°C gehalten, auf 100°C abgekühlt, mit 1930 g Wasser verdünnt, bei 80°C unter Einhaltung eines pH-Wertes von 1 bis 1,5 mit etwa 800 g 50 gew.-%iger Schwefelsäure angesäuert. Zum vollständigen Entfernen des im Hydrolysegemisch noch enthaltenen Schwefeldioxids wird das Gemisch 30 Minuten unter Einleiten von Stickstoff auf 80°C erwärmt. Anschliessend wird das Gemisch durch Verdampfungskühlung auf 50°C abgekühlt und 2 Stunden bei 50°C nachgerührt.

Das ausgefallene Produkt, das gemischte Mononatrium-Monokalium-Salz der H-Säure wird bei 50°C abfiltriert und mit insgesamt 200 g 15 gew.-%iger wässriger Natriumsulfatlösung gewaschen.

Ausbeute: 180 g feuchtes Produkt.

Die Zusammensetzung des feuchten Produktes wird durch Hochdruckflüssigkeitschromatographie bestimmt; das Produkt weist folgende Zusammensetzung auf (die angegebenen Gewichtsprozente beziehen sich auf die freien Säuren):

37,3 Gew.-% H-Säure
 0,6 Gew.-% K-Säure
 0   Gew.-% Iso-K-Säure (1-Amino-6-naphthol-4,8-disulfonsäure)

0 Gew.-% W-Säure (1-Amino-6-naphthol-3,8-disulfonsäure)

0 Gew.-% T-Säure (1-Naphtylamin-3,6,8-trisulfonsäure)

0,2 Gew.-% Chromotrop-Säure (1,8-Dihydroxynaphthalin-3,6-disulfonsäure)

0 Gew.-% Dioxy-K-Säure (1,8-Dihydroxynaphthalin-4,6-disulfonsäure)

Rest bis 100 Gew.-%: Wasser, Sulfat-, Natrium- und Kaliumionen.

Die Ausbeute an H-Säure beträgt 21% der Theorie, bezogen auf den Nitritgehalt, bzw. 71% der Theorie, bezogen auf den T-Säure-Gehalt des in die Druckhydrolyse eingesetzten Naphthylamin-trisulfonsäure-Gemisches.

Zur Reinigung werden die 180 g feuchtes Rohprodukt in 180 g Wasser suspendiert, die Suspension 1 Stunde auf 80°C erwärmt, auf 40°C abgekühlt und 2 Stunden bei 40°C nachgerührt. Das ausgefallene Produkt wird bei 40°C abfiltriert, mit insgesamt 75 g Wasser gewaschen und im Vakuum bei 60°C getrocknet.

Die Ausbeute beträgt (bei Wiederverwendung des Filtrates und des Waschwassers im nächsten Ansatz) 81 g trockenes Produkt; das sind 70% der Theorie, bezogen auf den T-Säuregehalt des in die Druckhydrolyse eingesetzten Naphthylaminstrisulfonsäuregemisches.

Die Reinheit der H-Säure wird durch Hochdruckflüssigkeitschromatographie bestimmt; die Zusammensetzung des trockenen Produktes beträgt (die angegebenen Gewichtsprozente beziehen sich auf freie Säuren):

81,9 Gew.-% H-Säure

0,3 Gew.-% K-Säure

0 Gew.-% Iso-K-Säure

0 Gew.-% W-Säure

0 Gew.-% T-Säure

0,2 Gew.-% Chromotrop-Säure

0 Gew.-% Dioxy-K-Säure

Das Produkt erhält ausserdem

10,0 Gew.-% Wasser

4,5 Gew.-% Kalium

3,0 Gew.-% Natrium

0,2 Gew.-% Sulfat

Das nach dem Abfiltrieren des rohen gemischten Mononatrium-Monokalium-Salzes der H-Säure angefallene Filtrat (4280 g) wird bei 50°C mit 428 g Natriumsulfat versetzt, auf Raumtemperatur (20°C) abgekühlt und 12 Stunden bei Raumtemperatur (20°C) gehalten.

Das ausgeallene K-Säuremononatriumsalz wird abfiltriert, mit insgesamt 300 g 15 gew.-%iger wässriger Natriumsulfatlösung gewaschen und im Vakuum bei 60°C getrocknet.

Die Ausbeute beträgt 194 g trockenes Produkt, das sind 35,5% der Theorie, bezogen auf den Nitritgehalt, bzw. 71% der Theorie, bezogen auf den Melansäuregehalt, des eingesetzten Naphthylamin-trisulfonsäuregemisches.

Die Reinheit der K-Säure wird durch Hochdruckflüssigkeitschromatographie bestimmt; die K-Säure weist folgende Zusammensetzung auf (die angegebenen Gewichtsprozente beziehen sich auf freie Säuren):

58,3 Gew.-% K-Säure

0 Gew.-% H-Säure

1,2 Gew.-% Iso-K-Säure

0 Gew.-% W-Säure

0 Gew.-% Melansäure

0 Gew.-% T-Säure

1,0 Gew.-% Dioxy-K-Säure

0 Gew.-% Chromotropsäure

Rest bis 100 Gew.-%: Wasser, Natrium- und Sulfationen.

Beispiel 2

Es wird wie in Beispiel 1 beschrieben verfahren, jedoch mit der Abänderung, dass das nach dem Abfiltrieren der Roh-H-Säure anfallende Filtrat zum Ausfällen der K-Säure bei 60°C mit 10 g Natriumchlorid je 100 g Filtrat versetzt wird. Das ausgefallene K-Säuremononatriumsalz wird nach dem Abfiltrieren mit insgesamt 300 g gesättigter Natriumchlorid-Lösung gewaschen.

Die Ausbeute an Roh-K-Säure beträgt 37,5% der Theorie, bezogen auf den Nitritgehalt bzw. 75% der Theorie, bezogen auf den Melansäuregehalt des in die Druckhydrolyse eingesetzten Naphthylamintrisulfonsäuregemisches.

Die Reinheit der K-Säure wird durch Hochdruckflüssigkeitschromatographie bestimmt; die Zusammensetzung der K-Säure beträgt (die angegebenen Gewichtsprozente beziehen sich auf die freien Säuren):

62,7 Gew.-% K-Säure

0 Gew.-% H-Säure

3,5 Gew.-% Iso-K-Säure

0,6 Gew.-% Dioxy-K-Säure

Rest bis 100 Gew.-%: Wasser, Natrium- und Chloridionen.

Zur Reinigung werden 150 g Rohprodukt in 150 g Wasser suspendiert, die Suspension auf 80°C erwärmt, auf 20°C abgekühlt und 2 Stunden bei 20°C nachgerührt. Das Produkt wird abfiltriert, mit insgesamt 75 g kaltem Wasser gewaschen und im Vakuum bei 60°C getrocknet.

Die Ausbeute beträgt (bei Rückführung des nach dem Abfiltrieren der K-Säure anfallenden Filtrates und des beim Waschen angefallenen Waschwassers) 112 g trockenes Produkt; das sind 73% der Theorie, bezogen auf den Melansäuregehalt des eingesetzten Naphthylamintrisulfonsäuregemisches.

Die Reinheit der gereinigten K-Säure wird durch Hochdruckflüssigkeitschromatographie bestimmt; die Zusammensetzung der K-Säure beträgt (die angegebenen Gewichtsprozente beziehen sich auf die freien Säuren):

81,4 Gew.-% K-Säure

0 Gew.-% H-Säure

0,05 Gew.-% Iso-K-Säure

0,1 Gew.-% Dioxy-K-Säure

Das Produkt enthält ausserdem

14,0 Gew.-% Wasser
3,7 Gew.-% Natrium
0,9 Gew.-% Chlorid
110   ppm Kalium

Beispiel 3
740 g Naphthylamin-trisulfonsäure-Gemisch in Form der Trinatriumsalze (Gehalt 9,33 g Gesamt-Nitrit/100 g, 26,6 Gew.-% Melansäure MG 383; insgesamt 69 g Nitrit, 0,51 Mol Melansäure) folgender Zusammensetzung:

| | |
|---|---|
| 1-Naphthylamin-4,6,8-trisulfonsäure | 51,4% |
| 1-Naphthylamin-3,6,8-trisulfonsäure | 30,9% |
| 2-Naphthylamin-4,6,8-trisulfonsäure | 9,0% |
| 1-Naphthylamin-2,5,7-trisulfonsäure | 1,2% |
| 1-Naphthylamin-3,5,7-trisulfonsäure | 2,5% |
| 2-Naphthylamin-3,6,8-trisulfonsäure | 0,2% |

(%-Gehalte jeweils bezogen auf diazotierbare Substanz); das zusätzlich 8,1 Gew.-% Wasser und quantitativ nicht bestimmbare Mengen an weiteren Nebenprodukten enthält, sowie 260 g (6,5 Mol) Natriumhydroxid und 547 g Wasser werden, wie in Beispiel 1 beschrieben, umgesetzt.

Nach dem Verdünnen des Hydrolysegemisches mit 1453 g Wasser lässt man die alkalische Reaktionslösung gleichzeitig mit etwa 900 g 50 gew.-%iger Schwefelsäure bei 80°C unter Einhaltung eines pH-Wertes von 1 bis 1,5 unter Rühren in einen Reaktionskolben einlaufen, in dem eine Mischung aus 261 g (1,5 Mol) Kaliumsulfat und 500 ml Wasser vorgelegt war.

Die Aufarbeitung der so erhaltenen sauren Lösung erfolgt wie in Beispiel 1 beschrieben. H-Säure und K-Säure werden in gleichen Ausbeuten und Qualitäten erhalten wie in Beispiel 1.

Beispiel 4
673 g Naphthylamin-trisulfonsäure-[trinatriumsalz]-Gemisch (insgesamt 69 g Nitrit, 0,72 Mol Melansäure) folgender Zusammensetzung:

| | | |
|---|---|---|
| 72,3% Melansäure | | |
| 16,8% T-Säure | | |
| 1,6% 1-Naphthylamin-3,5,7-trisulfonsäure | bezogen auf diazotierbare Substanz | |
| 6,5% 2-Naphthylamin-4,6,8-trisulfonsäure | | |

werden mit 120 g (3,0 Mol) Natriumhydroxid, 196 g (3,5 Mol) Kaliumhydroxid und 682 g Wasser wie in Beispiel 1 beschrieben umgesetzt und wie folgt aufgearbeitet:

Isolierung der H-Säure
1671 g der alkalischen, Natriumsulfit-haltigen Reaktionsmischung (Suspension) werden mit 1529 g Wasser verdünnt.
3200 g dieser klaren verdünnten alkalischen Reaktionslösung werden gleichzeitig mit 1020 g 50 gew.-%iger Schwefelsäure bei 80°C unter Einhaltung eines pH-Wertes von 1 bis 1,5 innerhalb 1 Stunde in einen 800 g Wasser enthaltenden Siebenhalskolben gegeben.

Das gebildete Schwefeldioxid wird durch 30-minütiges Einleiten von Stickstoff bei 80°C ausgetrieben. Die Reaktionsmischung (Suspension) wird auf 50°C abgekühlt und 2 Stunden bei 50°C nachgerührt. Anschliessend wird die ausgefallene H-Säure abfiltriert und 2mal mit je 75 g gesättigter Natriumsulfatlösung gewaschen.

Auf diese Weise werden 95 g feuchter Roh-H-Säure erhalten.

Isolierung der K-Säure
Das nach dem Abfiltrieren der Roh-H-Säure angefallene Filtrat und die beim Auswaschen der Roh-H-Säure anfallende Waschlösung werden vereinigt und bei 50°C mit etwa 500 g Kochsalz (10 g Kochsalz/100 g Filtrat) unter Rühren versetzt. Die Mischung wird auf 20°C abgekühlt und etwa 12 Stunden bei 20°C nachgerührt. Anschliessend wird die ausgefallene K-Säure abfiltriert, 2mal mit je 250 g gesättigter Kochsalzlösung gewaschen und im Vakuum bei 80°C getrocknet.

Es werden erhalten etwa 326 g rohe trockene K-Säure.

Die Ausbeute an technisch reiner, trockener K-Säure beträgt 54,3% der Theorie, bezogen auf den Nitritgehalt bzw. 75% der Theorie, bezogen auf den Melansäuregehalt des in die Druckhydrolyse eingesetzten Naphthylamintrisulfonsäuregemisches.

Der Reinigungsgrad der K-Säure wird ebenfalls durch Hochdruckflüssigkeitschromatographie bestimmt; die K-Säure weist folgende Zusammensetzung auf:

| | |
|---|---|
| 53,1 Gew.-% K-Säure | |
| 1,0 Gew.-% H-Säure | |
| 3,7 Gew.-% Iso-K-Säure | |
| 0,3 Gew.-% Dioxy-K-Säure | |
| 0,1 Gew.-% Chromotropsäure | |
| 0 Gew.-% Melansäure | |
| 0 Gew.-% T-Säure ferner | |
| 27 Gew.-% Kochsalz | |
| 6 Gew.-% Wasser. | |

Reinigung der Roh-H-Säure
95 g rohe feuchte H-Säure werden in etwa 150 g Wasser angeschlämmt. Die Mischung, die einen pH-Wert von 1 bis 1,5 aufweist, wird auf 80°C erwärmt und 1 Stunde bei dieser Temperatur gehalten, dann auf 40°C abgekühlt und 2 Stunden bei dieser Temperatur nachgerührt. Anschliessend wird die H-Säure abfiltriert, und nacheinander mit 25 g Eiswasser gewaschen. Anschliessend wird die Säure im Vakuum bei 80°C getrocknet. Es werden erhalten 45,8 g reine trockene H-Säure entsprechend einer Ausbeute von 11,3% der Theorie, bezogen auf den Gesamtnitritgehalt bzw. 67% der Theorie, bezogen auf den T-Säuregehalt des eingesetzten Naphthylamin-trisulfonsäuregemisches.

Der Reinheitsgrad der H-Säure wird durch Hochdruckflüssigkeitschromatographie bestimmt; die Zusammensetzung der H-Säure beträgt:

78,4 Gew.-% H-Säure
0,7 Gew.-% K-Säure
0    Gew.-% Iso-K-Säure
0    Gew.-% W-Säure
0    Gew.-% T-Säure
0    Gew.-% Chromotropsäure
0    Gew.-% Dioxy-K-Säure
     ferner
0,6 Gew.-% Sulfat
11,7 Gew.-% Wasser
3,0 Gew.-% Natrium
4,8 Gew.-% Kalium.

## Patentansprüche

1) Verfahren zur Isolierung von H-Säure und K-Säure in Form ihrer Monoalkalisalze aus diese beiden Säuren, Alkaliionen und gegebenenfalls andere Amino-naphthol-disulfonsäuren enthaltenden sauren wässrigen Lösungen durch Ausfällen der H-Säure-Monoalkalisalze bei höheren Temperaturen und Ausfällen der K-Säure-Monoalkalialze durch Abkühlen des nach dem Abtrennen der H-Säure anfallenden Filtrates, dadurch gekennzeichnet, dass man die H-Säure aus Lösungen ausfällt, die sowohl Natrium-als auch Kalium-ionen enthalten.

2) Verfahren gemäss Anspruch 1, dadurch gekennzeichnet, dass das Verhältnis von Natriumionen zu Kaliumionen in den Lösungen 5–1:1 beträgt.

3) Verfahren gemäss Ansprüchen 1 und 2, dadurch gekennzeichnet, dass das Verhältnis Natriumionen zu Kaliumionen in den Lösungen 3–2:1 beträgt.

4) Verfahren gemäss Ansprüchen 1 bis 3, dadurch gekennzeichnet, dass die Gesamtkonzentration an Alkaliionen in den sauren Lösungen zum Ausfällen der H-Säure 1 bis 10 g-Atome/l Lösung beträgt.

5) Verfahren gemäss Ansprüchen 1 bis 4, dadurch gekennzeichnet, dass die Konzentration an Natriumionen in dem nach dem Abfiltrieren der H-Säure anfallenden Filtrat zum Ausfällen der K-Säure 1 bis 10 g-Atome/l Filtrat beträgt.

6) Verfahren gemäss Ansprüchen 1 bis 5, dadurch gekennzeichnet, dass der pH-Wert der sauren Lösungen zum Ausfällen der H-Säure 0 bis 4 beträgt.

## Claims

1) Process for isolating H acid and K acid in the form of their monoalkali metal salts from acid aqueous solutions which contain these two acids and alkali metal ions and which may contain other aminonaphtholdisulphonic acids, by precipitating H acid monoalkali metal salts at elevated temperatures and precipitating K acid monoalkali metal salts by cooling down the filtrate obtained on separating off the H acid, characterised in that the H acid is precipitated from solutions which contain not only sodium ions but also potassium ions.

2) Process according to Claim 1, characterised in that in the solutions the ratio of sodium ions to potassium ions is 5–1:1.

3) Process according to Claims 1 and 2, characterised in that in the solutions the ratio of sodium ions to potassium ions is 3–2:1.

4) Process according to Claims 1 to 3, characterised in that to precipitate the H acid the total concentration of alkali metal ions in the acid solutions is 1 to 10 g atoms/l of solution.

5) Process according to Claims 1 to 4, characterised in that to precipitate K acid the concentration of sodium ions in the filtrate obtained on filtering off H acid is 1 to 10 g atoms/l of filtrate.

6) Process according to Claims 1 to 5, characterised in that to precipitate H acid the pH value of the acid solutions is 0 to 4.

## Revendications

1. Procédé pour isoler l'acide H et l'acide K sous la forme de leurs sels mono-alcalins de solutions acides aqueuses contenant ces deux acides, des ions alcalins et, le cas échéant, d'autres acides aminonaphtoldisulfoniques par précipitation des sels mono-alcalins de l'acide H à températures élevées et précipitation des sels mono-alcalins d'acide K par refroidissement du filtrat obtenu après séparation de l'acide H, caractérisé en ce qu'on précipite l'acide H dans des solutions qui contiennent aussi bien des ions sodium que des ions potassium.

2. Procédé suivant la revendication 1, caractérisé en ce que le rapport des ions sodium aux ions potassium dans les solutions s'élève à 5–1:1.

3. Procédé suivant les revendications 1 et 2, caractérisé en ce que le rapport des ions sodium aux ions potassium dans les solutions s'élève à 3–2:1.

4. Procédé suivant les revendications 1 à 3, caractérisé en ce que la concentration totale en ions alcalins dans les solutions acides utilisées pour la précipitation de l'acide H va de 1 à 10 atomes-g/l de solution.

5. Procédé suivant les revendications 1 à 4, caractérisé en ce que la concentration en ions sodium dans le filtrat obtenu après séparation par filtration de l'acide H pour la précipitation de l'acide K a une valeur de 1 à 10 atomes-g/l de filtrat.

6. Procédé suivant les revendications 1 à 5, caractérisé en ce que la valeur de pH des solutions acides pour la précipitation de l'acide H va de 0 à 4.